# EUROPÄISCHE PATENTSCHRIFT

(11) **EP 1 025 835 B1**
(45) Veröffentlichungstag und Bekanntmachung des Hinweises auf die Patenterteilung: **23.03.2005**
(21) Anmeldenummer: 00250030.4
(22) Anmeldetag: 31.01.2000
(51) Int. Cl.: A61K 7/40, A61K 7/48

(54) **Kosmetische Zubereitung zum Schutz der Kopfhaut vor freien Radikalen**
Cosmetic composition for protecting the scalp from free radicals
Composition cosmétique pour la protection du cuir chevelu contre des radicaux libres

(30) Priorität: 01.02.1999 DE 19905127
(43) Veröffentlichungstag der Anmeldung: 09.08.2000
(73) Patentinhaber: Coty B.V., 2031 CC Haarlem (NL)
(72) Erfinder: Herrling, Thomas, Dr., 13187 Berlin (DE); Groth, Norbert, 12625 Waldesruh (DE); Golz-Berner, Karin, 98000 Monaco (MC); Zastrow, Leonhard, 98000 Monaco (MC)
(74) Vertreter: Walter, Wolf-Jürgen

(56) Entgegenhaltungen:
- WO-A-92/19224
- WO-A-96/29048
- WO-A-99/66881
- DE-A- 19 830 004
- DE-A- 19 849 228
- PATENT ABSTRACTS OF JAPAN vol. 010, no. 161 (C-352), 10. Juni 1986 (1986-06-10) & JP 61 015845 A (HISAMITSU SEIYAKU KK), 23. Januar 1986 (1986-01-23)

## Beschreibung

Die Erfindung betrifft eine kosmetische Zubereitung zum Schutz der Kopfhaut vor freien Radikalen und die Verwendung der Zubereitung.

Es ist bekannt, daß Haarfärbemittel erhebliche Anteile von Wasserstoffperoxid enthalten, da allgemein zuerst ein Entfärbungsprozeß mit Hilfe von Wasserstoffperoxid (H₂O₂) und dann ein Färbungsprozeß mittels der farbgebenden Komponente erfolgt.

H₂O₂ ist bekannt hinsichtlich seiner Fähigkeit, zusammen mit Eisen- und/oder Kupferionen das äußerst reaktionsfähige Hydroxyradikal OH^{≅} zu bilden. Eisen und Kupfer sind teilweise in Haarfärbemitteln enthalten; Eisen ist immer im Blut und damit in winzigen Hautwunden präsent. Das Hydroxyradikal ist in der Lage, Strukturen des menschlichen Körpers zu attackieren und dabei Zellmembranen, Enzyme oder die DNS zu zerstören. Auf diese Weise spielen sie eine große Rolle bei der Entstehung zahlreicher Krankheiten, und es existieren Anzeichen, daß freie Radikale auch den Alterungsprozeß beeinflussen.

In der WO 99/66881 wurde im Beispiel 3 ein Sonnengel vorgeschlagen, das neben einem radikalfangenden Wirkstoffkomplex 1 Gew-% einer wässrigen Schallacklösüng enthält.

Aus JP 61-015845 A (Abstract) ist eine Zusammensetzung für die Epidermis mit Freisetzungseigenschaften für Arzeimittelkomponenten bekannt, die in Alkohol gelösten Schellack enthält.

Es ist weiterhin bekannt, vitaminhaltige Zubereitungen mit Chitosanen (DE 198 49 228), Superoxid-dismutase mit Phosphonsäurederivaten (WO 92/19224) und pflanzliche und tierische Abbauprodukte kondensiert mit Fettsäurehalogeniden (WO 96/29048) als kosmetische Mittel auf die Haut aufzubringen.

Der Erfindung liegt die Aufgabe zugrunde, die Bildung von Hydroxyradikalen auf der Kopfhaut weitgehend zu verhindern und gebildete Radikale zu reduzieren. Erfindungsgemäß wird diese Aufgabe durch eine wäßrige Dispersion, Emulsion oder ein wäßriges Gel gelöst, das eine radikalfangende Komponente auf Basis enzymatischer Strukturen mit einem Anteil von 0,5 bis 30 Gew-%, einen wasserlöslichen oder wasserdispergierbaren Filmbildner für die Haut auf Basis von Schellack oder Dextrinen mit einem Anteil von 0,1 bis 20 Gew-%, Wasser mit einem restlichen Anteil bis 100 Gew-% und sonstige kosmetische Hilfs- und Trägerstoffe mit einem Anteil von 0 bis 55 Gew-% enthält. Der Schellackgehalt in der Schellacklösung oder -dispersion liegt bei 5 bis 60 Gew-%.

Es wurde gefunden, daß insbesondere enzymatische Radikalfänger am schnellsten in der Lage sind, gebildete Hydroxyradikale mit einer Halbwertzeit von t_{1/2} = 1 ns und einer mittleren Weglänge, bezogen auf die Halbwertzeit, von ΔX_{1/2} = 2nm zu erreichen, gegebenenfalls zu eliminieren und vor allem die von den Hydroxyradikalen hervorgerufenen Kettenreaktionen zu unterbrechen. Durchaus Einfluß hat dabei auch die Konzentration der Radikalfänger, da sie in unmittelbarer Nachbarschaft des Entstehungsortes der radikalischen Energie vorhanden sein sollten. Da der Radikalfänger die radikalische Energie schneller als andere Moleküle absorbieren muß, muß er auch etwa in der gleichen Dimension wie die Radikalbildungskonstante liegen. Dies wird mit der erfindungsgemäßen Zubereitung erreicht. Dabei übernimmt der kosmetische Filmbildner für die Haut die Funktion, einen wesentlichen Teil des H₂O₂ von der Haut durch die feine Filmbildung auf der Haut abzuschirmen.

Als Filmbildner für die kosmetische Zubereitung können bevorzugt eingesetzt werden wäßrige Schellacklösungen oder -dispersionen, bestehend aus
(a) Schellack-Trockensubstanz,
(b) einem wasserlöslichem Filmbildner innerhalb der Schellacklösung oder -dispersion, ausgewählt unter Polyvinylpyrrolidon, Chitosan, mikrokristallinem Chitosan, quarterniertem Chitosan, Polymeren der Acrylsäurerreih2e, quarternären Cellulosederivaten, Hyaluronsäure und deren Salzen sowie Gemischen davon,
(c) einem im sauren Bereich beständiger Gelbildner, wie ein modifiziertes natürliches Polysaccharid, wie Guar Hyroxypropyltrimonium Chloride, Agar, Alginate, Alginsäure; sowie Gemische davon,
(d) einer Säure zur pH-Wert-Einstellung und
(e) Wasser.

Derartige Schellacklösungen oder -dispersionen mit einem Schellackgehalt von 5 bis 60 Gew-% werden hergestellt durch Mischen von reiner Schellack-Trockensubstanz mit einem Molekulargewicht von etwa 1000 mit Wasser in Gegenwart von einem wasserlöslichen Filmbildner im Bereich von 0,1 bis 3 Gew-% und einem im sauren Bereich beständigen Gelbildner im Bereich von 0,1 bis 1 Gew-% und einer säure zur Einstellung des pH-Wertes auf einen Wert von 2 - 4,2 unter Rühren bei 150 bis 1000 U/Min während eines Zeitraumes von 5 bis 60 Minuten bei 20 bis 45 °C. Diese Lösungsen sind bei Kontakt mit dem enzymatischen Radikalfänger sehr stabil und können somit einen wirksamen Schutz gegen H₂O₂ gewährleisten.

Ein weiterer Filmbildner im Zusammenhang mit dem Radikalfänger sind Dextrine, wie α-, β- und γ-Dextrin, allein oder vorteilhaft als Gemisch. Bei einem Dextringemisch wird ein Verhältnis α:β:γ im Bereich von 2,5-1,9:1:2,5-1,9 bevorzugt.

Weitere mögliche Filmbildner sind Dimethiconolol Behenate, Hydrogenated Polyisobutene, Adipic Acid/Diethylene Glycol/Glycerine Crosspolymer, Eicosene Copolymer, Tricontanyl PVP, PPG-12/SMDI Copolymer, PPG-8/SMDI Copolymer, Cyclomethicone & Dimethicone, Cetyl Dimethicone, Cyclomethicone & Dimethiconol, Tridecyl Stearate & Tridecyl Trimellitate & Dipentaerithryl Hexacaprylate/Hexacaprate.

Es können Gemische von Schellack und Dextrin(en) als Filmbildner eingesetzt werden, die eine überraschende Verbesserung der filmbildenden Eigenschaften zeigen. Das Verhältnis Schellack:Dextrin(e) liegt im Bereich von 10-1:1, vorzugsweise im Bereich 5-2:1.

Der Anteil des Filmbildners in der erfindungsgemäßen Zubereitung liegt vorteilhaft im Bereich von 5 bis 20 Gew-%, vorzugsweise 10 bis 20 Gew-%.

Bevorzugt sind Filmbildner, die den Film auf der Haut ausbilden und nicht oder nur zum geringen Teil auf keratinhaltigen Oberflächen.

Die radikalfangende Komponente enthält insbesondere enzymatische Strukturen aus Hefen oder Pflanzen.

Derartige radikalfangende Komponenten mit enzymatischen Strukturen sind vorteilhaft ausgewählt unter
(a) einem wäßrigen Wirkstoff, bestehend aus einem Produkt
   (a1) das durch Extraktion der Rinde von Quebracho blanco und nachfolgender enzymatischer Hydrolyse gewonnene ist und das wenigstens 90 Gew- % Proanthocyanidin-Oligomere und höchstens 10 Gew- % Gallussäure enthält, wobei der Gehalt von (a1), das in einer an Mikrokapseln gebundenen Wirkstoffkonzentration von 2 Gew- % vorliegt, im Bereich von 1 bis 10 Gew- % liegt;
   (a2) einem durch Extraktion gewonnenen Seidenraupenextrakt, der das Peptid Cecropine, Aminosäuren und ein Vitamingemisch enthält, wobei der Gehalt von (a2) im Bereich von 0,1 bis 10 Gew-% liegt;
   (a3) einem nichtionischen, kationischen oder anionischen Hydro-Gel oder Gemisch von Hydro-Gelen mit einem Gehalt von (a3) im Bereich von 0,1 bis 5 Gew-%;
   (a4) einem oder mehreren Phospholipiden mit einem Anteil von 0,1 bis 30 Gew-%;
   (a5) Wasser
   (a6) einem wenigstens 150 Einheiten Superoxiddismutase pro ml enthaltenden Ultraschall-Aufschlußprodukt einer Hefe, wobei der Gehalt des Aufschlußproduktes (a6) im Bereich von 0 bis 4 Gew-% liegt; und
   (a7) einem Extrakt von Acerolafrüchten Malpighia punidifolia, wobei der Gehalt (a7) im Bereich von 0 bis 30 Gew-% liegt; jeweils bezogen auf das Gesamtgewicht des wäßrigen Wirkstoffes;
(b) einem Wirkstoff, bestehend aus
   (b1) einem Produkt eines enzymatischen Extraktionsprozesses des maritimen Planktons Artemia salina, wobei das Extraktionsprodukt aus phosphorylierten Nucleotiden mit der Hauptkomponente Diguanosin-tetraphosphat besteht;
   (b2) D-myo-Inosit-1,4,5-triphosphat;
   (b3) Glycan; wobei das Verhältnis b1:b2:b3 im Bereich 1 : 0,1-50 : 0,1-30 liegt;
(c) einem Wirkstoff gemäß WO96/29048, bestehend aus dem Produkt eines direkten und milden Abbaus bei 20 - 55 °C und einem pH-Wert von 7,5 - 8,5 eines biologischen Ausgangsmaterials im wäßrigen Medium, einer nachfolgenden Kondensation mit einer unterstöchiometrischen Menge eines C₁₀-C₂₀-Fettsäurehalogenids oder eines C₁₀-C₂₀-Fettsäurehalogenidgemisches und gegebenenfalls einer nachfolgenden Veretherung oder Veresterung des Kondensationsproduktes, wobei das Ausgangsmaterial Hefen, Hefefraktionen, Hülsenfrüchten, Hülsenfrüchtefraktionen, Pektine, pektinhaltige Massen, Algen, Algenfraktionen, Tiermilch, Tiermilchfraktionen und deren Gemische umfaßt;
(d) einem Wirkstoff, hergestellt durch ein Aufschlußverfahren gemäß EP 626997 von Hefen, wie Bäckerhefe, Brauereihefe, Weinhefe und Superoxiddismutase-angereicherte Hefen mittels Ultraschall, bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht und wobei die Sonotrode einen Winkel von 80,5 bis 88,5° zur gegenüberliegenden Ebene des Beschallungsraumes hat, und das Verhältnis der Eintauchlänge der Sonotrode in mm zum Beschallungsvolumen in ml auf einen Wert von 1:1,1 bis 1:20 eingestellt wird.

Eine besonders bevorzugte radikalfangende Komponente ist der oben unter (a) aufgeführte Wirkstoff, der einen hohen Radikalschutzfaktor von 100 bis 3500 aufweisen kann.

Der Extrakt von (a1) und von (a2) ist als Produkt unter dem Namen Magnalys bzw. Radicalys erhältlich von Greentech, Saint-Beauzire, Frankreich.

Das Produkt unter (b1) ist als GP4G kommerziell erhältlich von Laboratoires Seporga, Sophia-Antipolis, Frankreich.

Der Anteil der radikalfangenden Komponente liegt vorzugsweise im Bereich von 10 bis 30 Gew-%, insbesondere im Bereich von 20 bis 30 Gew-%.

In einer Ausführungsform kann der Anteil der radikalfangenden Komponente im Bereich von 5 bis 25 Gew-% liegen.

Die erfindungsgemäße Zubereitung kann als Emulsion, Dispersion als Gel vorliegen, gegebenenfalls in Kombination mit waschaktiven Mitteln.

Generell vorteilhaft sind dünnflüssige Zubereitungen wegen der besseren Verteilbarkeit auf der Haut. Es können auch Sprays hergestellt werden. Um eine möglichst hohe antiradikalische Wirksamkeit zu entwickeln, kann die erfindungsgemäße Zubereitung auch in Ampullen abgefüllt und luftdicht verschlossen einer Haarfärbemittelpackung beigegeben werden, um unmittelbar vor Gebrauch erst mit Luft und dann mit der Kopfhaut in Kontakt gebracht zu werden. Bekanntlich unterliegen Radikalfänger generell Autoxidationsprozessen, die auf diese Weise erfolgreich gehemmt werden können.

Als zusätzliche Radikalfänger weiterhin möglich sind Vitamine wie Vitamin C und Derivate davon, beispielsweise Ascorbylacetat, -phosphat und -palmitat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Trypophan und Derivate davon; Carotinoide und Carotine, wie z.B α-Carotin, β-Carotin; Harnsäure und Derivate davon. Mit diesen Radikalfängern sind die schnellen Reaktionen mit den OH^{•}-Radikalen jedoch nicht immer gewährleistet.

. Bevorzugt sind die Radikalfänger lipophil, um möglicherweise auch in die Kopfhaut einzudringen und dort die Reaktion mit vorhandenen OH^{•}-Radikalen einzugehen. Es können jedoch auch hydrophile Radikalfänger verwendet werden, da das Vorhandensein auf der Kopfhaut und deren Abschirmung eine sehr wichtige Aufgabe dieser Stoffe ist. Auch Gemische hydrophiler und lipophiler Radikalfänger können vorteilhaft sein.

Die Erfindung betrifft auch die Verwendung der kosmetischen Zubereitung als wäßrige Dispersion, Emulsion oder Gel, bestehend aus einer enzymatische Strukturen umfassenden radikalfangenden Komponente, einem wasserlöslichen oder wasserdispergierbaren Filmbildner auf Basis von Schellack, Dextrinen oder Gemischen davon für die Haut, Wasser und sonstige kosmetische Hilfs- und Trägerstoffen, zum Schutz der Kopfhaut vor freien Radikalen.

Bevorzugt wird unmittelbar vor dem Auftragen von Wasserstoffperoxid oder andere radikalbildende Stoffe enthaltenden Haarfärbemitteln die Dispersion, Emulsion oder das Gel auf die Kopfhaut durch Einreiben oder Einsprühen aufgetragen.

Die Entfernung der erfindungsgemäßen Zubereitungen von der Kopfhaut erfolgt allgemein durch einen Waschvorgang, der üblicherweise dem Färbeprozeß nachgeschaltet ist.

Die Erfindung soll nachstehend durch Beispiele näher erläutert werden. Alle Angaben erfolgen in Gewichtsprozent, sofern nichts anderes angegeben ist.

| Beispiel 1 **Kopfhautspray** | |
|---|---|
| α Dextrine | 5,0 |
| β Dextrine | 2,5 |
| γ Dextrine | 5,0 |
| Radikalfängerkomplex* | 30,0 |
| Konservierungsmittel | 0,5 |
| Wasser | ad 100 |

| | |
|---|---|
| * Der Radikalfängerkomplex aus Quebracho blanco und Seidenraupenextrakt wurde wie folgt hergestellt: | |

Zur Herstellung der Gelgrundlage wurde Gelpulver, wie Carbomer, in Wasser gegeben, darin homogenisiert und anschließend z.B. mit Triethanolamin neutralisiert. Danach erfolgte eine Zugabe von Ethanol und Glycerin zur besseren Einarbeitbarkeit, wobei die entstehende Mischung gut verrührt wurde. Zu dieser Gelgrundlage wurde ein Gemisch von Phospholipiden (Phoslipon®), Quebracho-Extrakt und Seidenraupenextrakt gegeben und bei einer Temperatur von höchstens 45 °C damit viermischt. Anschließend erfolgte die Zugabe eines weiteren Teiles des obigen Gels oder eines zweiten Gels, wie Guar Propyltriammoniumchlorid, das gut mit dem Gesamtgemisch bei erhöhter Temperatur jedoch unter 45 °C verrührt wurde. Danach erfolgte die Zugabe von Acerolaextrakt und gegebenenfalls Vitamin C und Vitamin D (Acetat).

Der Komplex enthielt 1% trockenes Gel, 7% Phospholipide, 2% Quebracho-Extrakt, 1% Seidenraupen-Extrakt, 1 % Acerolaextrakt und zusätzlich jeweils 0,5 % Vitamin C und Vitamin A.

Die Komponenten von Beispiel 1 wurden wie folgt verarbeitet.

Die einzelnen Dextrine wurden nacheinander in Wasser bei Raumtemperatur eingerührt. Danach wurde der Radikalfängerkomplex dann das Konservierungsmittel zugegeben und solange gerührt, bis kein Rückstand mehr optisch wahrnehmbar war. Die erhaltene Dispersion wurde in eine Sprühflasche abgefüllt.

| | |
|---|---|
| Beispiel 2 **Kopfhautgel** | |
| Carbomer | 1,5 |
| Triethanolamin | 1,5 |
| Shellac | 20 |
| Antaron (Tricontange PVP) | 3 |
| Radikalfängerkomplex | 25,0 |
| Konservierungsmittel | 0,5 |
| Wasser | ad 100 |

Der Gelbildner Carbomer wurde in Wasser eingerührt und homogenisiert. Danach erfolgte eine Neutralisation mit TEA. Dann wurde Schellack in Form einer wäßrige Schellacklösung mit 18 Gew-% Schellack, bezogen auf die Lösung, unter Rühren zugegeben, dann Antaron und schließlich der Radikalfängerkomplex gemäß Beispiel 1 (Quebrachoextrakt 5 % und ohne Zusätze von Vitaminen) und das Konservierungsmittel. Das Gemisch wurde homogen verrührt.

| Beispiel 3 **Sprühbare Kopfhautlotion I** | |
|---|---|
| **Phase A** | |
| Ceteareth 12 | 4,0 |
| Glyceryl Stearate | 1,5 |
| Hydrogenated Castor Oil Laurate | 7,0 |
| Soy Sterol | 0,5 |
| | |
| **Phase B** | |
| Wasser | ad 100 |
| **Phase C** | |
| Konservierungsmittel | 0,5 |
| **Phase D** | |
| Radikalfängerkomplex | 28,0 |
| **Phase E** | |
| Shellac | 10 |
| PPG-8/SmdI Copolymer | 2,0 |

Phase A und B wurden auf 80 bis 85 °C erhitzt und Phase B in Phase A homogenisiert. Nach Abkühlen auf 25 °C erfolgte die Zugabe von Phase C, D (gemäß Beispiel 1 aber ohne Vitaminzusatz und ohne Acerolaextrakt) und E (20 Gew-% Schellack, bezogen auf die Schellacklösung) nacheinander zu dem Anfangsgemisch, und das Gesamtgemisch wurde gut verrührt.

### Beispiel 4 Sprühbare Kopfhautlotion II

Die Zusammensetzung entsprach der von Beispiel 3, mit Ausnahme des Radikalfängers, für den 25 % eines Hefe-Abbauproduktes mit nachfolgender Fettsäurehalogenid-Kondensation gemäß Beispiel 1 von WO96/29048 eingesetzt wurden, und mit Ausnahme des Fimbildners, für den 9 % eines 3:1-Gemisches aus Schellack (20%-ige Lösung) und δ-Dextrin (20%ige Lösung) eingesetzt wurden.

## Patentansprüche

1. Kosmetische Zubereitung zum Schutze der Kopfhaut vor freien Radikalen, **gekennzeichnet durch**
eine wäßrige Dispersion, Emulsion oder ein wäßriges Gel, enthaltend eine aus enzymatischen Strukturen bestehende radikalfangende Komponente mit einem Anteil von 0,5 bis 30 Gew-%, einen wasserlöslichen oder wasserdispergierbaren Filmbildner für die Haut mit einem Anteil von 5 bis 20 Gew-%, Wasser mit einem restlichen Anteil bis 100 Gew-% und sonstige kosmetische Hilfs- und Trägerstoffe mit einem Anteil von 0 bis 55 Gew-%, bezogen auf die Gesamtzusammensetzung, wobei der Filmbildner ausgewählt ist unter einer wäßrigen Schellacklösung oder
- dispersion mit einem Schellackgehalt von 5-60 Gew-%, bezogen auf die Schellacklösung oder -dispersion, einem Dextrin, einem Dextringemisch oder einem Gemisch von Schellack und Dextrin(en), und wobei die wäßrige Schellacklösung oder - dispersion aus
(a) Schellack-Trockensubstanz,
(b) einem wasserlöslichem Filmbildner innerhalb der Schellacklösung oder -dispersion, ausgewählt unter Polyvinylpyrrolidon, Chitosan, mikrokristallinem Chitosan, quarterniertem Chitosan, Polymeren der Acrylsäurerreihe, quarternären Cellulosederivaten, Hyaluronsäure und deren Salzen sowie Gemischen davon,
(c) einem im sauren Bereich beständiger Gelbildner, wie ein modifiziertes natürliches Polysaccharid wie Guar Hyroxypropyltrimonium Chloride, Agar, Alginate, Alginsäure; sowie Gemische davon,
(d) einer Säure zur pH-Wert-Einstellung und
(e) Wasser besteht.

2. Zubereitung nach Anspruch 1, **dadurch gekennzeichnet, daß** die enzymatischen Strukturen aus Hefen oder Pflanzen herrühren.

3. Zubereitung nach Anspruch 2, **dadurch gekennzeichnet, daß** die radikalfangende Komponente ausgewählt ist unter
(a) einem wäßrigen Wirkstoff, bestehend aus einem Produkt
(a1) das durch Extraktion der Rinde von Quebracho blanco und nachfolgender enzymatischer Hydrolyse gewonnene ist und das wenigstens 90 Gew- % Proanthocyanidin-Oligomere und höchstens 10 Gew- % Gallussäure enthält, wobei der Gehalt von (a1), das in einer an Mikrokapseln gebundenen Wirkstoffkonzentration von 2 Gew- % vorliegt, im Bereich von 1 bis 10 Gew- % liegt;
(a2) einem durch Extraktion gewonnenen Seidenraupenextrakt, der das Peptid Cecropine, Aminosäuren und ein Vitamingemisch enthält, wobei der Gehalt von (a2) im Bereich von 0,1 bis 10 Gew- % liegt;
(a3) einem nichtionischen, kationischen oder anionischen Hydro-Gel oder Gemisch von Hydro-Gelen mit einem Gehalt von (a3) im Bereich von 0,1 bis 5 Gew-%;
(a4) einem oder mehreren Phospholipiden mit einem Anteil von 0,1 bis 30 Gew-%;
(a5) Wasser
(a6) einem wenigstens 150 Einheiten Superoxiddismutase pro ml enthaltenden Ultraschall-Aufschlußprodukt einer Hefe, wobei der Gehalt des Aufschlußproduktes (a6) im Bereich von 0 bis 4 Gew-% liegt; und
(a7) einem Extrakt von Acerolafrüchten Malpighia punidifolia, wobei der Gehalt (a7) im Bereich von 0 bis 30 Gew-% liegt; jeweils bezogen auf das Gesamtgewicht des wäßrigen Wirkstoffes;
(b) einem Wirkstoff, bestehend aus
(b1) einem Produkt eines enzymatischen Extraktionsprozesses des maritimen Planktons Artemia salina, wobei das Extraktionsprodukt aus phosphorylierten Nucleotiden mit der Hauptkomponente Diguanosin-tetraphosphat besteht;
(b2) D-myo-Inosit-1,4,5-triphosphat;
(b3) Glycan; wobei das Verhältnis b1:b2:b3 im Bereich 1 : 0,1-50 : 0,1-30 liegt;
(c) einem Wirkstoff, bestehend aus dem Produkt eines direkten und milden Abbaus bei 20 - 55 EC und einem pH-Wert von 7,5 - 8,5 eines biologischen Ausgangsmaterials im wäßrigen Medium, einer nachfolgenden Kondensation mit einer unterstöchiometrischen Menge eines C₁₀-C₂₀-Fettsäurehalogenids oder eines C₁₀-C₂₀-Fettsäurehalogenidgemisches und gegebenenfalls einer nachfolgenden Veretherung oder Veresterung des Kondensationsproduktes, wobei das Ausgangsmaterial Hefen, Hefefraktionen, Hülsenfrüchten, Hülsenfrüchtefraktionen, Pektine, pektinhaltige Massen, Algen, Algenfraktionen, Tiermilch, Tiermilchfraktionen und deren Gemische umfaßt;
(d) einen Wirkstoff, hergestellt durch ein Aufschlußverfahren von Hefen, wie Bäckerhefe, Brauereihefe, Weinhefe und Superoxiddismutase-angereicherte Hefen mittels Ultraschall, bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht und wobei die Sonotrode einen Winkel von 80,5 bis 88,5E zur gegenüberliegenden Ebene des Beschallungsraumes hat und das Verhältnis der Eintauchlänge der Sonotrode in mm zum Beschallungsvolumen in ml auf einen Wert von 1:1,1 bis 1:20 eingestellt wird.

4. Zubereitung nach einem der Ansprüche 1 bis 3, **dadurch gekennzeichnet, daß** der Anteil der radikalfangenden Komponente im Bereich von 10 bis 30 Gew-% liegt, insbesondere im Bereich von 20 bis 30 Gew-%.

5. Zubereitung nach Anspruch 3, **dadurch gekennzeichnet, daß** der Anteil der radikalfangenden Komponente im Bereich von 5 bis 25 Gew-% liegt.

6. Zubereitung nach Anspruch 1, **dadurch gekennzeichnet, daß** der Anteil des Filmbildners im Bereich von 10 bis 20 Gew-% liegt.

7. Zubereitung nach Anspruch 1, **dadurch gekennzeichnet, daß** die Schellacklösung oder -dispersion eine wäßrige Lösung oder Dispersion mit einem Schellackgehalt von 5 bis 60 Gew-% ist, hergestellt durch Mischen von reiner Schellack-Trockensubstanz mit einem Molekulargewicht von etwa 1000 mit Wasser in Gegenwart von einem wasserlöslichen Filmbildner im Bereich von 0,1 bis 3 Gew-% und einem im sauren Bereich beständiden Geibildner im Bereich von 0,1 bis 1 Gew-% und einer Säure zur Einstellung des pH-Wertes auf einen Wert von 2 - 4,2 unter Rühren bei 150 bis 1000 U/Min während eines Zeitraumes von 5 bis 60 Minuten bei 20 bis 45 EC.

8. Verwendung einer kosmetischen Zubereitung als wäßrige Dispersion, Emulsion oder Gel, bestehend aus einer aus enzymatischen Strukturen bestehenden radikalfangenden Komponente, einem wasserlöslichen oder wasserdispergierbaren Filmbildner für die Haut auf Basis von Schellacklösungen oder - dispersionen oder Dextrinen oder Gemischen davon, Wasser und sonstige kosmetische Hilfs- und Trägerstoffen, zum Schutz der Kopfhaut vor freien Radikalen.

9. Verwendung nach Anspruch 9, **dadurch gekennzeichnet, daß** unmittelbar vor dem Auftragen von Wasserstoffperoxid oder andere radikalbildende Stoffe enthaltenden Haarfärbemitteln die Dispersion, Emulsion oder das Gel auf die Kopfhaut durch Einreiben oder Einsprühen aufgetragen wird.

## Claims

1. A cosmetic composition for protecting the scalp from free radicals, comprising
an aqueous dispersion, emulsion or an aqueous gel containing a free-radical-scavenging component consisting of enzymatic structures, which component makes up 0.5 to 30% by weight, a water-soluble or water-dispersible film-forming agent for the skin making up 5 to 20% by weight, water making up the remainder up to 100% by weight, and other cosmetic auxiliaries and carriers marking up 0 to 55% by weight, relative to the overall composition, said film-forming agent being selected from among an aqueous shellac solution or dispersion whose shellac content is 5-60% by weight relative to the shellac solution or dispersion, a dextrin, a dextrin mixture or a mixture of shellac and dextrin (s), and said aqueous shellac solution or dispersion consisting of
(a) shellac dry substance,
(b) a water-soluble film-forming agent included in said shellac solution or dispersion, selected from among polyvinylpyrrolidone, chitosan, microcrystalline chitosan, quaternized chitosan, polymers of the acrylic acid series, quaternary cellulose derivatives, hyaluronic acid and salts thereof, and mixtures of the foregoing,
(c) a gel-forming agent which is stable in the acid range, such as a modified natural polysaccharide such as Guar Hydroxypropyltrimonium Chloride, Agar, Alginate, alginic acid; and mixtures thereof,
(d) an acid for adjusting the pH value, and
(e) water.

2. A composition according to claim 1, wherein the enzymatic structures stem from yeasts or plants.

3. A composition according to claim 2, wherein the free-radical-scavenging component is selected from among
(a) an aqueous active agent consisting of a product
(a1) obtained by extracting the bark of Quebracho blanco and subsequent enzymatic hydrolysis, which product contains at least 90% by weight proanthocyanidine oligomers and max. 10% by weight gallic acid, (a1), whose active agent concentration is 2% by weight, bound to microcapsules, making up between 1 and 10% by weight;
(a2) a silkworm extract obtained by extraction, which extract contains the peptide cecropine, amino acids and a vitamin mixture (a2) making up between 0.1 and 10% by weight;
a3) a non-ionic cationic or anionic hydrogel or hydrogel mixture, (a3) making up between 0.1 and 5% by weight;
(a4) one or several phospholipid(s) making up between 0.1 and 30% by weight;
(a5) water;
(a6) an ultrasonic decomposition product of a yeast, which decomposition product contains at least 150 units superoxide dismutase per ml, (a6) making up between 0 and 4% by weight;
(a7) an extract from acerola fruits (Malpighia punidifolia), (a7) making up between 0 and 30% by weight; each of the aforesaid percentages being relative to the total weight of the aqueous active agent;
(b) an active agent consisting of
(b1) a product obtained by an enzymatic extraction process of the maritime plankton Artemia salina, which extraction product consists of phosphorylated nucleotides whose main component is diguanosine tetraphosphate;
(b2) D-myo-inositol-1,4,5-triphosphate;
(b3) glycan; the ratio of b1 : b2 : b3 being in the range of 1 : 0.1-50 : 0.1-30;
(c) an active agent consisting of a product obtained by a direct and gentle decomposition of a biological starting material at 20-55° C and a pH value of 7.5-8.5 in an aqueous medium, a subsequent condensation with a substoichiometric amount of a C₁₀-C₂₀ fatty acid halogenide or a C₁₀-C₂₀ fatty acid halogenide mixture and, optionally, a subsequent etherification or esterification of the condensation product, said starting material comprising yeasts, yeast fractions, pulses, pulse fractions, pectins, masses containing pectin, algae, algae fractions, animal milk, animal milk fractions and mixtures thereof;
(d) an active agent produced by means of an ultrasonic decomposition process of yeasts such as baker's yeast, brewer's yeast, wine yeast and yeasts enriched with superoxide dismutase, in which process a cell dispersion or suspension is passed through an ultrasonic exposure area in an ultrasonic flow-through cell, in which the sonotrode extends into the flow-through cell up to half or two thirds of its length and is immersed in the medium to be exposed to ultrasound, the sonotrode being arranged at an angle ranging between 80.5 and 88.5° relative to the opposite plane of the ultrasonic exposure area and the ratio of the sonotrode's immersed length in mm to the volume exposed to ultrasound in ml being adjusted to a value ranging between 1:1.1 and 1:20.

4. A composition according to any one of claims 1 to 3, wherein the free-radical-scavenging component makes up between 10 and 30% by weight, particularly 20 and 30% by weight.

5. A composition according to claim 3, wherein the free-radical-scavenging component makes up between 5 and 25% by weight.

6. A composition according to claim 1, wherein the film-forming agent makes up between 10 and 20% by weight.

7. A composition according to claim 1, wherein the shellac solution or dispersion is an aqueous solution or dispersion whose shellac content is 5-60% by weight and which is prepared by mixing pure shellac dry substance having a molecular weight of approx. 1,000 with water in the presence of a water-soluble film-forming agent making up between 0.1 and 3% by weight and a gel-forming agent which is stable in the acid range and makes up 0.1 to 1% by weight and an acid for adjusting the pH value to a value between 2 and 4.2 while stirring at 150-1,000 rpm for 5-60 minutes at 20-45° C.

8. The use of a cosmetic composition as an aqueous dispersion, emulsion or gel, consisting of a free-radical-scavenging component consisting of enzymatic structures, a water-soluble or water-dispersible film-forming agent for the skin based on shellac solutions or dispersions or dextrins or mixtures thereof, water and other cosmetic auxiliaries and carriers for protecting the scalp from free radicals.

9. The use according to claim 9, wherein the dispersion, emulsion or gel is rubbed or sprayed onto the scalp immediately before hair dyes containing hydrogen peroxide or other free-radical-forming substances are applied.

## Revendications

1. Composition cosmétique pour la protection du cuir chevelu contre les radicaux libres, **caractérisée par**
une dispersion, émulsion aqueuse ou un gel aqueux, contenant un composant piégeant les radicaux, consistant en des structures enzymatiques, en une proportion de 0,5 à 30% en poids, un agent filmogène pour la peau, soluble dans l'eau ou dispersible dans l'eau, en une proportion de 5 à 20% en poids, de l'eau en une proportion résiduelle de jusqu'à 100% en poids et certains auxiliaires et supports cosmétiques en une proportion de 0 à 55% en poids, sur base de la composition totale, où l'agent filmogène est choisi parmi une solution ou une dispersion aqueuse de gomme-laque, avec une teneur en gomme-laque de 5-60% en poids, sur base de la solution ou dispersion de gomme-laque, une dextrine, un mélange de dextrine ou un mélange de gomme-laque et de dextrine(s), et où la solution ou dispersion aqueuse de gomme-laque consiste en
a) une substance sèche gomme-laque,
b) un agent filmogène soluble dans l'eau dans la solution ou dispersion de gomme-laque, choisi parmi la polyvinylpyrrolidone, le chitosan, le chitosan microcristallin, le chitosan quaternisé, des polymères de la série de l'acide acrylique, des dérivés quaternaires de la cellulose, l'acide hyaluronique et ses sels, ainsi que leurs mélanges,
(c) un agent formant gel stable en milieu acide, comme un polysaccharide naturel modifié comme le gomme de guar, le chlorure d'hydroxypropyltrimonium, l'agarose, un alginate, l'acide alginique ;
(d) un acide pour ajuster le pH, et
(e) de l'eau.

2. Composition selon la revendication 1, **caractérisée en ce que** les structures enzymatiques proviennent de levures ou de plantes.

3. Composition selon la revendication 2, **caractérisée en ce que** le composant piégeant les radicaux est choisi parmi
(a) une substance active aqueuse, consistant en un produit
(a1) qui est obtenu par extraction d'écorce de Quebracho blanco et hydrolyse enzymatique suivante, et qui contient au moins 90% en poids d'oligomères de proanthocyanidine et au maximum 10% en poids d'acide gallique, où la teneur en (a1), qui est présent en une concentration en agent actif lié à des microcapsules, de 2% en poids, se situe dans l'intervalle allant de 1 à 10% en poids ;
(a2) un extrait de pépins de raisin obtenu par extraction, qui contient le peptide cécropine, des acides aminés et un mélange de vitamines, où la teneur en (a2) se situe dans l'intervalle allant de 0,1 à 10% en poids ;
(a3) un hydrogel non ionique, cationique ou anionique, ou un mélange d'hydrogels, avec une teneur en (a3) située dans l'intervalle allant de 0,1 à 5% en poids ;
(a4) un ou plusieurs phospholipides avec une proportion de 0,1 à 30% en poids ;
(a5) de l'eau
(a6) un produit de désintégration aux ultra-sons contenant au moins 150 unités de superoxyde dismutase par ml, d'une levure, où la teneur en produit de désintégration (a6) se situe dans l'intervalle allant de 0 à 4% en poids, et
(a7) un extrait de fruits d'acérola Malpighia punidifolia, on la teneur en (a7) se situe dans l'intervalle allant de 0 à 30% en poids, chaque fois sur base du poids total des agents actifs aqueux ;
(b) un agent actif, consistant en
(b1) un produit d'un processus d'extraction enzymatique du plancton maritime Artemia salina, où le produit d'extraction consiste en des nucléotides phosphorylés avec le composant principal tétraphosphate de diguanosine ;
(b2) le 1,4,5-triphosphate de D-myoinositol ;
(b3) un glycane, où le rapport b1:b2:b3 se situe dans l'intervalle 1:0,1-50:0,1-30 ;
(c) un agent actif, consistant en le produit d'une dégradation directe et douce à 20-55°C et à un pH de 7,5-8,5, d'un matériau de départ biologique en milieu aqueux, d'une condensation suivante avec une quantité sous-stoechiométrique d'un halogénure d'acide gras en C₁₀-C₂₀ ou d'un mélange d'halogénures d'acide gras en C₁₀-C₂₀ et le cas échéant, une éthérification ou estérification ultérieure du produit de condensation, où le matériau de départ comprend des levures, des fractions de levure, des légumes secs, des fractions de légumes secs, la pectine, des masses contenant de la pectine, des algues, des fractions d'algues, du lait animal, des fractions de lait animal et leurs mélanges ;
(d) un agent actif, préparé par un procédé de désintégration par ultra-sons, de levures, comme la levure de boulangerie, la levure de brasserie, la levure de vin et des levures enrichies en superoxyde dismutase, où une dispersion ou suspension de cellules est conduite dans une chambre de sonication dans une cellule de circulation à ultra-sons , où le sonotrode plonge de la moitié aux deux-tiers de sa longueur dans la cellule de circulation et est immergé dans le milieu à soniquer et où le sonotrode a un angle de 80,5 à 88,5° par rapport au plan opposé à la chambre de sonication et le rapport de la longueur immergée du sonotrode en mm au volume de sonication en ml est ajusté à une valeur de 1:1,1 à 1:20.

4. Composition selon une des revendications 1 à 3, **caractérisée en ce que** la proportion du composant piégeant les radicaux se situe dans l'intervalle allant de 10 à 30% en poids, en particulier dans l'intervalle allant de 20 à 30% en poids.

5. Composition selon la revendication 3, **caractérisée en ce que** la proportion du composant piégeant les radicaux se situe dans l'intervalle allant de 5 à 25% en poids

6. Composition selon la revendication 1, **caractérisée en ce que** la proportion de l'agent filmogène se situe dans l'intervalle allant de 10 à 20% en poids

7. Composition selon la revendication 1, **caractérisée en ce que** la solution ou dispersion de gomme-laque est une solution ou dispersion aqueuse avec une teneur en gomme-laque de 5 à 60% en poids, préparée par mélange d'une substance sèche de gomme-laque pure avec un poids moléculaire d'environ 1000, avec de l'eau en présence d'un agent filmogène soluble dans l'eau, dans l'intervalle de 0,1 à 3% en poids et d'un agent formant gel stable en milieu acide, dans l'intervalle allant de 0,1 à 1% en poids et d'un acide pour ajuster le pH à une valeur de 2-4,2, sous agitation de 150 à 1000 Tours/minute pendant une période de 5 à 60 minutes de 20 à 45°C.

8. Utilisation d'une composition cosmétique sous la forme d'une dispersion, émulsion ou gel aqueux, consistant en un composant piégeant les radicaux consistant en des structures enzymatiques, un agent filmogène pour la peau soluble dans l'eau ou dispersible dans l'eau, à base de solutions ou de dispersions de gomme-laque ou de dextrines ou de mélanges de ceux-ci, d'eau et de certains auxiliaires et support cosmétiques, pour la protection du cuir chevelu contre les radicaux libres.

9. Utilisation selon la revendication 8, **caractérisée en ce que** directement avant l'application du peroxyde d'hydrogène ou d'autres colorants capillaires contenant des substances formant radicaux, on applique la dispersion, émulsion ou le gel sur le cuir chevelu, par enduction ou pulvérisation.
